# EUROPEAN PATENT APPLICATION

(11) **EP 1 743 645 A1**
(43) Date of publication of application: **17.01.2007**
(21) Application number: 05745877.0
(22) Date of filing: 31.05.2005
(51) Int. Cl.: A61K 31/506, A61K 9/70, A61K 47/12, A61K 47/14, A61K 47/16, A61K 47/32, A61P 25/22, A61P 25/24, A61P 43/00

(54) **ADHESIVE PATCH**

(30) Priority: 01.06.2004 JP 2004163709
(71) Applicant: HISAMITSU PHARMACEUTICAL CO., INC., Tosu-shi, Saga 841-0017 (JP)
(72) Inventor: YAMAGUCHI, Toshiro, HISAMITSU PHARMACEUT. CO. INC., Tsukuba-shi, Ibaraki 3050856 (JP); HIGO, Naruhito Hisamitsu Pharmaceutical Co., Inc., Tosu-shi, Saga 841-0017 (JP)
(74) Representative: Grünecker, Kinkeldey, Stockmair & Schwanhäusser Anwaltssozietät
(86) International application number: PCT/JP2005/009994
(87) International publication number: WO 2005/117886

(57) **Abstract**

The object of the invention is to provide a patch that contains tandospirone or a pharmaceutically acceptable salt thereof as the drug component, and has a sufficiently high percutaneous absorption rate. A patch according to a preferred embodiment of the invention comprises a backing layer and a pressure-sensitive adhesive layer formed on one side of the backing layer, wherein the pressure-sensitive adhesive layer contains a drug component composed of tandospirone and/or a pharmaceutically acceptable salt thereof at 5 to 30 wt% based on the weight of the pressure-sensitive adhesive layer, an acrylic-based polymer and/or styrene-based block copolymer, and an organic acid and/or a salt thereof.

## Description

### Technical Field

The present invention relates to a patch. More specifically, the invention relates to a patch which comprises tandospirone or its salt as a drug component and which is useful for treatment of psychosomatic disorders and the like.

### Background Art

Tandospirone ((1R,2S,3R,4S)-N-[4-{4-(pyrimidin-2-yl)piperazin-1-yl}butyl]-2,3-bicyclo[2,2,1]heptanedicarboxyimide) and its salts are non-benzodiazepine compounds with serotonergic activity, and they are known to be useful as antianxiety agents (for example, see Patent document 1). Tandospirone is believed to exhibit its antianxiety effect and ameliorating effect in psychosomatic disorder models by selective action on the 5-HTIA receptor, an intracerebral serotonin receptor subtype. Tandospirone is also predicted to have an antidepressant effect by lowering the density of serotonin neuroterminal post-synaptic 5-HT2 receptors.

When administered orally, however, tandospirone has low bioavailability and a short action time. Percutaneous absorbing preparations containing tandospirone have therefore been proposed, whereby metabolite production is suppressed and tandospirone blood levels are maintained for prolonged periods (for example, see Patent document 2).
[Patent document 1] Japanese Examined Patent Publication HEI No. 1-28756
[Patent document 2] International Patent Publication No. 97/37659
[Patent document 3] Japanese Unexamined Patent Publication HEI No. 11-228414

### Disclosure of the Invention

### Problems to be Solved by the Invention

Percutaneous administration using percutaneous absorbing preparations such as conventional patches can provide the advantages of long duration of action, reduced frequency of administration and improved compliance, but the percutaneous absorption rate is not always satisfactory. The stratum corneum of normal skin is highly lipophilic and functions as a barrier preventing infiltration of foreign substances into the body, but in the case of conventional percutaneous absorbing preparations, the barrier function has made it difficult to achieve percutaneous absorption of drug components at a satisfactory rate. A low percutaneous absorption rate tends to lengthen the time required from initial administration until the drug component reaches a sufficiently high level in the blood.

It is therefore an object of the present invention to provide a patch that contains tandospirone or a pharmaceutically acceptable salt thereof as the drug component, and has a sufficiently high percutaneous absorption rate of the drug component.

### Means for Solving the Problems

In order to achieve this object, the patch of the invention is characterized by comprising a backing layer and a pressure-sensitive adhesive layer formed on one side of the backing layer, wherein the pressure-sensitive adhesive layer contains: a drug component composed of tandospirone and/or a pharmaceutically acceptable salt thereof at 5 to 30 wt% based on the weight of the pressure-sensitive adhesive layer; an acrylic-based polymer and/or styrene-based block copolymer; and an organic acid and/or a salt thereof.

By employing a pressure-sensitive adhesive layer comprising a drug component composed of tandospirone and/or a pharmaceutically acceptable salt thereof in the aforementioned specific proportion, in combination with the components specified above, the patch of the invention can exhibit a satisfactorily increased percutaneous absorption rate. The present inventors found that by combining these components in the pressure-sensitive adhesive layer containing tandospirone as the drug component, it is possible to specifically improve the percutaneous absorption rate of the drug component. In the pressure-sensitive adhesive layer of the tandospirone-containing patch of the invention, the tandospirone is present as tandospirone alone, as a salt with the said organic acid and/or as a different salt, and the organic acid is present as the organic acid alone, as a salt with tandospirone and/or as another salt. In other words, at least part of the tandospirone and at least part of the organic acid in the pressure-sensitive adhesive layer may form pharmaceutically acceptable salts in the pressure-sensitive adhesive layer.

At least part of the drug component is preferably crystallized. Crystallization of the drug component can increase the content of the drug component while minimizing skin irritation. If the drug component is crystallized, presumably the concentration of the drug component dissolved in the pressure-sensitive adhesive layer is lowered, thereby reducing skin irritation. Generally speaking, an increased drug component content tends to result in a higher percutaneous absorption rate and a longer duration of action, but increasing the content of highly crystalline compounds such as tandospirone has conventionally caused crystallization in the pressure-sensitive adhesive layer of the patch, and it has been believed that such crystallization is the reason preventing satisfactory improvement in the percutaneous absorption rate even with an increased content. Techniques have therefore been developed, as in Patent document 2, for example, in which a recrystallization inhibitor such as an aliphatic alcohol is added to prevent crystallization of buspirone, and this document teaches that the percutaneous absorption rate achieved without addition of the recrystallization inhibitor is lower than with addition. However, in the case of a pressure-sensitive adhesive layer comprising a combination of the specific components mentioned above in a patch of the invention, the percutaneous absorption rate of the drug component content can be increased with higher content despite its crystallization, and therefore a high percutaneous absorption rate is achieved without addition of a recrystallization inhibitor, although the mechanism thereof is not yet fully understood. Moreover, using a recrystallization inhibitor as disclosed in Patent document 2 tends to increase skin irritation as a direct result of the recrystallization inhibitor, and therefore the present invention is superior in this regard as well.

The aforementioned styrene-based block copolymer is a block copolymer having blocks consisting of polystyrene chains. A preferred styrene-based block copolymer is styrene-isoprene-styrene block copolymer, from the standpoint of the percutaneous absorption rate of the drug component and cohesion of the pressure-sensitive adhesive layer.

Acetic acid is preferred as the organic acid. Using acetic acid can produce a more notable effect of percutaneous absorption rate increase.

The pressure-sensitive adhesive layer more preferably also contains a fatty acid ester and/or fatty acid amide. Including these components in the pressure-sensitive adhesive layer can produce a more notable effect of percutaneous absorption rate increase.

### Effect of the Invention

The present invention provides a patch that contains tandospirone or a pharmaceutically acceptable salt thereof as the drug component, and has a sufficiently high percutaneous absorption rate of the drug component.

### Brief Description of the Drawings

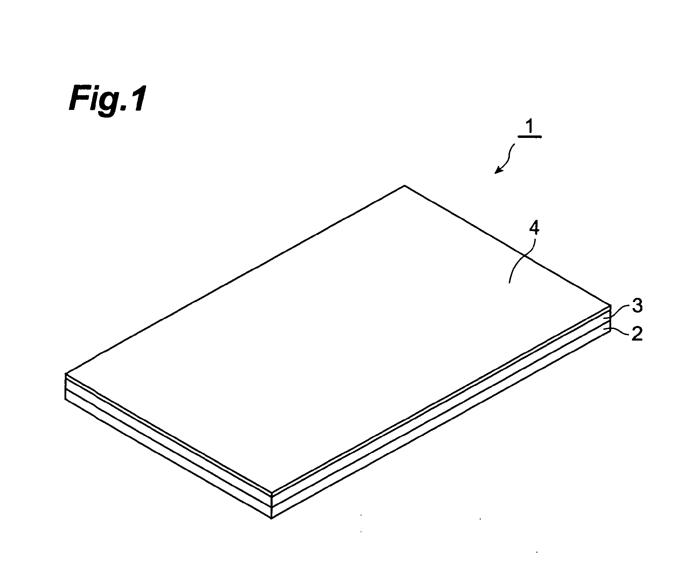

Fig. 1 is a perspective view of a preferred embodiment of the patch of the invention.

### Explanation of Symbols

1: Patch, 2: backing layer, 3: pressure-sensitive adhesive layer, 4: release liner

### Best Modes for Carrying Out the Invention

The patch of the invention comprises a backing layer and a pressure-sensitive adhesive layer formed on one side of the backing layer. The pressure-sensitive adhesive layer contains a drug component composed of tandospirone and/or a pharmaceutically acceptable salt thereof at 5 to 30 wt% based on the weight of the pressure-sensitive adhesive layer, an acrylic-based polymer and/or styrene-based block copolymer, and an organic acid and/or a salt thereof.

The tandospirone or its salt used as the drug component may be synthesized by a conventional publicly known process. The salt of tandospirone may be an inorganic salt or organic salt. As organic salts there may be mentioned organic acid salts such as citric acid salt, acetic acid salt, propionic acid salt, succinic acid salt, lactic acid salt, malic acid salt, tartaric acid salt, maleic acid salt, fumaric acid salt, methanesulfonic acid salt, p-toluenesulfonic acid salt and ascorbic acid salt. Particularly preferred among these organic salts are citric acid salt.

The drug component content is 5 to 30 wt% and more preferably 5 to 20 wt% based on the weight of the pressure-sensitive adhesive layer. A content of less than 5 wt% will result in an insufficient percutaneous absorption rate, whereas a content of greater than 30 wt% will increase skin irritation due to the drug component.

The drug component may be crystallized at least partially. The crystallized drug component may be used as the starting material with a portion thereof remaining in the pressure-sensitive adhesive layer, or the drug component may be first dissolved in another component and then a portion thereof crystallized (recrystallized). Here, "crystallized" means that the drug component is present in the pressure-sensitive adhesive layer in a crystalline state, and it includes cases where it has precipitated in a state that includes amorphous regions. Crystallization of the drug component can be confirmed by polariscopy of the pressure-sensitive adhesive layer with an optical microscope, or by naked eye observation. Since in most cases the refractive index of a phase containing drug component crystals differs from the refractive index of other phases in the pressure-sensitive adhesive layer, the pressure-sensitive adhesive layer will appear turbid when observed with the naked eye, thereby allowing crystallization of the drug component to be confirmed. Needle-like crystals of the drug component can also be confirmed visually in some cases.

The pressure-sensitive adhesive layer contains at least one of an acrylic-based polymer and styrene-based block copolymer. It is particularly preferred to use an acrylic-based polymer and styrene-based block copolymer in combination. Their use in combination is preferred in a proportion (styrene-based block copolymer:acrylic-based polymer) of 1:0.1 to 1:10 in terms of weight ratio. The combined content of the acrylic-based polymer and styrene-based block copolymer in the pressure-sensitive adhesive layer is preferably 10 to 50 wt% based on the total weight of the pressure-sensitive adhesive layer.

An acrylic-based polymer is a polymer having an acrylic acid ester or methacrylic acid ester monomer residue as the main monomer unit, and it may be a straight-chain polymer or may have a partially crosslinked structure. Other than a (meth)acrylic acid ester, the monomers of the acrylic-based polymer may include (meth)acrylamide, (meth)acrylic acid, vinylpyrrolidone, vinyl ester, vinyl alcohol or the like. The acrylic-based polymer is obtained by polymerization of these monomers alone or in combinations of two or more, or in combination with other copolymerizable monomers if necessary, by conventional publicly known processes.

As acrylic acid esters composing acrylic-based polymers there may be mentioned methyl acrylate, ethyl acrylate, propyl acrylate, amyl acrylate, butyl acrylate, 2-ethylbutyl acrylate, hexyl acrylate, heptyl acrylate, octyl acrylate, nonyl acrylate, 2-ethylhexyl acrylate, decyl acrylate, dodecyl acrylate, tridecyl acrylate, methoxyethyl acrylate, ethoxyethyl acrylate and the like, which may be used alone or in combinations of two or more.

More specifically, as acrylic-based polymers there may be mentioned (A1) block copolymers of polymethyl methacrylate with polyacrylates obtained by polymerization of one or more monomers selected from among 2-ethylhexyl acrylate, butyl acrylate, diacetoneacrylamide and tetraethyleneglycol dimethacrylate, (A2) copolymers of 2-ethylhexyl acrylate with N-vinyl-2-pyrrolidone and 1,6-hexaneglycol dimethacrylate, (A3) aminoalkyl methacrylate copolymer and (A4) 2-ethylhexyl acrylate and vinyl acetate copolymer, among which (A2) and (A4) are preferred for their especially high drug component percutaneous absorption rates and adhesive strengths.

As commercially available acrylic-based polymers there may be mentioned Duro-Tak 87-2097, Duro-Tak 87-2194 and Duro-Tak 87-4098 (product names) by National Starch & Chemical Co., which may be suitably used alone or in combinations.

A styrene-based block copolymer is a block copolymer having blocks composed of polystyrene chains, and as examples there may be mentioned binary systems comprising a polystyrene chain and another copolymer chain bonded thereto, and ternary block copolymers having two polystyrene chains sandwiching another copolymer chain. As monomers to be copolymerized with styrene there may be mentioned dienes such as isoprene and butadiene.

More specifically, as styrene-based block copolymers there may be mentioned styrene-isoprene-styrene block copolymer (SIS), styrene-butadiene-styrene block copolymer (SBS) and styrene-butadiene block copolymer, which may be used alone or in combinations of two or more. Of these, styrene-isoprene-styrene block copolymer (SIS) is preferred from the standpoint of drug component percutaneous absorption rate and shelf life of the patch. More preferred is a combination of SIS with the aforementioned (A2) and/or (A4) as the acrylic-based polymer.

As organic acids there may be mentioned aliphatic acids, aromatic carboxylic acids, alkylsulfonic acids and cholic acid derivatives, which may be used alone or in combinations of two or more. Aliphatic acids are preferred among these from the standpoint of efficiently increasing the percutaneous absorption rate.

As aliphatic acids there may be mentioned acetic acid, propionic acid, citric acid, isobutyric acid, caproic acid, caprylic acid, lactic acid, maleic acid, pyruvic acid, oxalic acid, succinic acid and tartaric acid, with acetic acid being especially preferred. As aromatic carboxylic acids there may be mentioned phthalic acid, salicylic acid, benzoic acid and acetylsalicylic acid, as alkylsulfonic acids there may be mentioned methanesulfonic acid, ethanesulfonic acid, propylsulfonic acid, butanesulfonic acid and polyoxyethylene alkylether sulfonic acid, and as cholic acid derivatives there may be mentioned dehydrocholic acid and the like.

As organic acid salts there may be mentioned alkali metal salts such as sodium salts of the aforementioned organic acids. Preferred is a combination of acetic acid and sodium acetate. At least a portion of the tandospirone and at least a portion of the organic acid in the pressure-sensitive adhesive layer may form an organic acid salt as a pharmaceutically acceptable salt of tandospirone, and at least a portion of the organic acid salt may also be included in the crystallized drug component.

There are no particular restrictions on the organic acid content, but it is preferably 0.5 to 5 mol, more preferably 0.5 to 4 mol, even more preferably 0.5 to 4.5 mol and yet more preferably 0.5 to 3 mol with respect to 1 mol of the drug component. The lower limits for these numerical ranges is more preferably 1.0 mol. If the proportion is less than 0.5 mol the percutaneous absorption rate will tend to be lower, and if it is greater than 5 mol the pressure-sensitive adhesive layer will tend to plasticize, resulting in reduced adhesive strength.

A tackifier may be added to the pressure-sensitive adhesive layer mainly for the purpose of improving the adhesive property. Addition of a tackifier is particularly preferred for a pressure-sensitive adhesive layer containing a styrene-based block copolymer. As tackifiers there may be mentioned, specifically, rosins and their derivatives (rosin glycerin esters, hydrogenated rosins, hydrogenated rosin glycerin esters, rosin pentaerythritol esters, and the like), alicyclic saturated hydrocarbon resins, aliphatic hydrocarbon resins, terpene resins, maleic acid resins, and the like. Preferred among these for use as tackifiers are one or more selected from the group consisting of hydrogenated rosin glycerin esters, aliphatic hydrocarbon resins, terpene resins and alicyclic saturated hydrocarbon resins. Most preferred among these tackifiers are alicyclic saturated hydrocarbon resins.

Alicyclic saturated hydrocarbon resins are available as commercial products such as ARKON P100 (Arakawa Chemical Industries, Ltd.), aliphatic hydrocarbon resins are available as commercially products such as QUINTONE B-170 (Zeon Corp.), and terpene resins are available as commercially products such as CLEARON P-125 (Yasuhara Chemical Co., Ltd.).

The tackifier content is not particularly restricted, but for a pressure-sensitive adhesive layer containing a styrene-based block copolymer, it is preferably 10 to 65 wt% based on the total pressure-sensitive adhesive layer. If the tackifier content is less than 10 wt% the adhesive force of the patch will tend to be reduced, and if it is greater than 65 wt% the adhesive force will be too strong, thereby increasing skin irritation. For a pressure-sensitive adhesive layer containing only an acrylic-based polymer instead of a styrene-based block copolymer and acrylic-based polymer, the tackifier content is preferably 0 to 50 wt% based on the total pressure-sensitive adhesive layer. If the content is greater than 50 wt%, the adhesive force will be too strong and will tend to increase skin irritation.

When an alicyclic saturated hydrocarbon resin is used as the tackifier, the preferred weight ratio between the total content of the acrylic-based polymer and styrene-based block copolymer and the tackifier content is preferably 1:1 to 1:3.

The pressure-sensitive adhesive layer may also contain at least one of a fatty acid ester and fatty acid amide to further accelerate percutaneous absorption. As fatty acid esters there may be mentioned ethylene glycol fatty acid esters, propylene glycol fatty acid esters (propylene glycol monolaurate, etc.), glycerin fatty acid esters, polyethylene glycol fatty acid esters, polyglycerin fatty acid esters, isopropyl myristate, sorbitan fatty acid esters, polyoxyethylene sorbitan fatty acid esters (polysorbates, etc.) and the like, and as fatty acid amides there may be mentioned lauric acid diethanolamide and the like. Particularly preferred for combination with tandospirone and its salts are isopropyl myristate and lauric acid diethanolamide.

There are no particular restrictions on the fatty acid ester and/or fatty acid amide content, but it is preferably 0.01 to 20 wt%, more preferably 0.05 to 10 wt% and even more preferably 0.1 to 5 wt% based on the total pressure-sensitive adhesive layer. The lower limit for these numerical ranges is preferably 0.5 wt% and more preferably 1.0 wt%. If the content is less than 0.01 wt% the drug component percutaneous absorption rate will tend to be lower, and if it is greater than 20 wt% the skin irritation will tend to be increased.

There may also be added to the pressure-sensitive adhesive layer, as appropriate, other components including drug components other than tandospirone, elastomers such as ethylene-vinyl acetate polymer, plasticizers such as liquid paraffin, aliphatic ethers, antioxidants, fillers, ultraviolet absorbers and the like.

The patch of the invention comprises the pressure-sensitive adhesive layer described above formed on one side of a backing layer. Fig. 1 is a perspective view of a preferred embodiment of a patch according to the invention. The patch 1 shown in Fig. 1 is constructed with a sheet-like backing layer 2, a pressure-sensitive adhesive layer 3 adhered onto one side of the backing layer 2, and a release liner 4 adhered onto the side of the pressure-sensitive adhesive layer 3 opposite the backing layer 2. The patch 1 is used by peeling off the release liner 4 and then attaching the pressure-sensitive adhesive layer 3 onto the skin of a patient by adhesion.

The backing layer 2 is not particularly restricted so long as it can backing layer the pressure-sensitive adhesive layer 3, and it may be elastic or non-elastic. As the backing layer 2 there may be mentioned films made of polyurethane, polyesters (polyethylene terephthalate, etc.), polypropylene, polyvinyl acetate, vinyl acetate copolymer, polyvinylidene chloride, polyethylene, nylon, acryl, cotton, rayon, acetate and the like, fiber sheets such as woven fabrics and nonwoven fabrics, foam sheets, and the like.

Particularly preferred among these for the substrate 2 from the standpoint of safety and drug effect are films made of polyesters, and especially films made of polyethylene terephthalate.

The release liner 4 may be a sheet that can be peeled off without causing undue deformation of the pressure-sensitive adhesive layer. As examples of materials for the release liner 4 there may be mentioned films made of polyesters (polyethylene terephthalate, etc.), polyolefin, polyvinyl chloride and polyvinylidene chloride, laminated paper composed of high-quality paper and polyolefin, and laminated paper composed of high-quality paper and polyester. The release liner 4 is preferably silicone-treated on the side which contacts with the pressure-sensitive adhesive layer 3, in order to facilitate peeling of the release liner 4 from the patch 1.

The pressure-sensitive adhesive layer 3 may be formed, for example, by mixing the aforementioned materials for the pressure-sensitive adhesive layer 3 to prepare a pressure-sensitive adhesive base, and coating the base onto the backing layer 2. The coating may be accomplished with heating if necessary to melt the pressure-sensitive adhesive base. Alternatively, a solvent such as toluene, hexane or an acetic acid ester may be added to the pressure-sensitive adhesive base, the mixture coated onto the backing layer 2, and the solvent removed to form the pressure-sensitive adhesive layer 3.

The thickness of the pressure-sensitive adhesive layer 3 is not particularly restricted but is preferably 20 to 200 µm. A thickness of less than 20 µm for the pressure-sensitive adhesive layer 3 will tend to lower the percutaneous absorption rate, while a thickness of greater than 200 µm will tend to promote a phenomenon wherein a portion of the pressure-sensitive adhesive layer remains attached to the skin upon peeling after attachment to the skin (pressure-sensitive adhesive residue).

The patch 1 can be obtained by laminating the release liner 4 onto the pressure-sensitive adhesive layer 3 formed on the backing layer 2. As the reverse method, the pressure-sensitive adhesive layer 3 may be formed on the release liner 4 and then laminated onto the backing layer 2 to obtain the patch 1.

The patch of the invention is not limited to the embodiment described above, and for example, two or more pressure-sensitive adhesive layers may be formed.

### EXAMPLES

The present invention will now be explained in greater detail by examples and comparative examples, with the understanding that the invention is not limited to the examples.

### Example 1

### Fabrication of patch

To a solution of styrene-isobutylene-styrene block copolymer (SIS) and an alicyclic saturated hydrocarbon resin (ARKON P100 (product name) by Arakawa Chemical Industries, Ltd.) in toluene there was added a mixture of tandospirone (molecular weight: 365.48), acetic acid (molecular weight: 60.05), sodium acetate (molecular weight: 82.03), liquid paraffin and an acrylic-based polymer (Duro-Tak 87-4098 (product name) by National Starch & Chemical Co.), and the mixture was kneaded to complete uniformity to prepare a pressure-sensitive adhesive base having the composition listed in Table 1 (wt% based on the total pressure-sensitive adhesive base (excluding toluene)). The prepared pressure-sensitive adhesive base was coated onto one side of a releasable release liner and heated at 70°C for 10 minutes to remove the toluene, to form a pressure-sensitive adhesive layer with the thickness shown in Table 1 on one side of the release liner. A backing layer was laminated and contact bonded onto the pressure-sensitive adhesive layer for adhesion of the pressure-sensitive adhesive layer and backing layer, to obtain a patch having the same construction as the patch 1 shown in Fig. 1, with a pressure-sensitive adhesive layer formed on one side of the backing layer.

### In vitro skin permeation test

Skin peeled from the back of a hairless mouse (female, 8 weeks old) was fitted in a flow-through cell with exterior circulation of 32°C warm water, with the dermis side on the receptor tank side, and a patch obtained in the manner described above was attached to the stratum corneum of the skin. Physiological saline was used as the receptor solution for circulation at a flow rate of 5 ml/hr while sampling the receptor solution every 2 hours up to 24 hours. The flow rate of the receptor solution was measured, and the drug concentration of the receptor solution sampled at each time period was measured by high-performance liquid chromatography. The measured values for the flow rate and drug concentration were used to calculate the drug permeation rate per hour, in order to determine the maximum value of the drug permeation rate per unit area of skin (Flux) and the time from start of the test at which maximum Flux was reached (Tmax). The results of Example 1 are shown in Table 1, together with the results of Examples 2 to 10 and Comparative Examples 1 and 2.

### Example 2

Patch fabrication and a skin permeation test were carried out in the same manner as Example 1, except that the composition of the pressure-sensitive adhesive base had the weight ratio listed in Table 1.

### Example 3

Fabrication of a patch and a skin permeation test were carried out in the same manner as Example 1, except that the composition of the pressure-sensitive adhesive base had the weight ratio listed in Table 1.

### Example 4

To a solution of styrene-isobutylene-styrene block copolymer (SIS) and an alicyclic saturated hydrocarbon resin (ARKON P100 (product name) by Arakawa Chemical Industries, Ltd.) in toluene there was added a mixture of tandospirone, acetic acid, sodium acetate, liquid paraffin, an acrylic-based polymer (Duro-Tak 87-4098 (product name) by National Starch & Chemical Co.) and isopropyl myristate (IPM), and the mixture was kneaded to complete uniformity to prepare a pressure-sensitive adhesive base having the composition listed in Table 1. The prepared pressure-sensitive adhesive base was used for patch fabrication and a skin permeation test in the same manner as Example 1.

### Example 5

Patch fabrication and a skin permeation test were carried out in the same manner as Example 4, except that the composition of the pressure-sensitive adhesive base had the weight ratio listed in Table 1.

### Example 6

Patch fabrication and a skin permeation test were carried out in the same manner as Example 4, except that tandospirone citrate (molecular weight: 575.62) was used instead of tandospirone, and the composition of the pressure-sensitive adhesive base had the weight ratio listed in Table 1.

### Example 7

Patch fabrication and a skin permeation test were carried out in the same manner as Example 4, except that lauric acid diethanolamide (LADA) was used instead of isopropyl myristate, and the composition of the pressure-sensitive adhesive base had the weight ratio listed in Table 1.

### Example 8

After combining tandospirone, acetic acid, sodium acetate and isopropyl myristate, there were added an acrylic-based polymer (Duro-Tak 87-4098 (product name) by National Starch & Chemical Co.) and ethyl acetate, and the mixture was kneaded to complete uniformity to prepare a pressure-sensitive adhesive base having the composition listed in Table 1. The prepared pressure-sensitive adhesive base was used for patch fabrication and a skin permeation test in the same manner as Example 1.

### Example 9

Patch fabrication and a skin permeation test were carried out in the same manner as Example 8, except that the composition of the pressure-sensitive adhesive base had the weight ratio listed in Table 1.

### Example 10

Patch fabrication and a skin permeation test were carried out in the same manner as Example 8, except that the composition of the pressure-sensitive adhesive base had the weight ratio listed in Table 1.

### Comparative Example 1

Patch fabrication and a skin permeation test were carried out in the same manner as Example 1, except that the composition of the pressure-sensitive adhesive base had the weight ratio listed in Table 1.

### Comparative Example 2

Patch fabrication and a skin permeation test were carried out in the same manner as Example 4, except that the composition of the pressure-sensitive adhesive base had the weight ratio listed in Table 1.

**Table 1**

| | Pressure-sensitive adhesive base composition (wt%) | | | | | | | | | Pressure-sensitive adhesive layer thickness (µm) | Skin permeation test | |
|---|---|---|---|---|---|---|---|---|---|---|---|---|
| | Tandospirone | Tandospirone citrate | SIS | Acrylic polymer | Acetic acid | Sodium acetate | Alicyclic saturated hydrocarbon resin | Liquid paraffin | Other | | Maximum flux value | Tmax |
| Ex. 1 | 5 | 0 | 16.1 | 10.0 | 2.4 | 2.2 | 56.4 | 8.1 | 0 | 70 | 6.2 | 17 |
| Ex. 2 | 10 | 0 | 14.2 | 10.0 | 4.7 | 4.3 | 49.7 | 7.1 | 0 | 70 | 13.4 | 19 |
| Ex. 3 | 15 | 0 | 11.2 | 10.0 | 7.1 | 6.5 | 39.1 | 11.2 | 0 | 90 | 28.9 | 21 |
| Ex.4 | 15 | 0 | 10.6 | 10.0 | 7.1 | 6.5 | 37.2 | 10.6 | IPM3% | 90 | 39.3 | 17 |
| Ex. 5 | 10 | 0 | 11.5 | 10.0 | 7.1 | 6.5 | 40.4 | 11.5 | IPM3% | 75 | 29.5 | 15 |
| Ex. 6 | 0 | 15 | 10.0 | 10.0 | 7.1 | 9.8 | 35.1 | 10.0 | IPM3% | 80 | 29.3 | 21 |
| Ex.7 | 10 | 0 | 11.5 | 10.0 | 7.1 | 6.5 | 40.4 | 11.5 | LADA 3% | 80 | 48.2 | 9 |
| Ex. 8 | 5 | 0 | 0 | 87.5 | 2.4 | 2.2 | 0 | 0 | IPM3% | 65 | 8.5 | 13 |
| Ex. 9 | 10 | 0 | 0 | 78.0 | 4.7 | 4.3 | 0 | 0 | IPM3% | 65 | 5.5 | 11 |
| Ex. 10 | 15 | 0 | 0 | 68.5 | 7.1 | 6.5 | 0 | 0 | IPM3% | 65 | 7.0 | 13 |
| Comp. Ex. 1 | 1 | 0 | 17.6 | 10.0 | 0.5 | 0.4 | 61.7 | 8.8 | 0 | 70 | 1.1 | 19 |
| Comp. Ex. 2 | 15 | 0 | 13.1 | 10.0 | 0 | 0 | 45.8 | 13.1 | IPM3% | 90 | 2.5 | 23 |

As shown in Table 1, the patches of Examples 1 to 10 exhibited larger maximum Flux values than Comparative Examples 1 and 2. It was thus confirmed that the invention provides a patch that contains tandospirone or a pharmaceutically acceptable salt thereof as the drug component and has a sufficiently high percutaneous absorption rate of the drug component.

### Industrial Applicability

The patch of the invention comprising tandospirone or its salt as a drug component can be used for treatment of psychosomatic disorders and the like.

## Claims

1. A patch comprising a backing layer and a pressure-sensitive adhesive layer formed on one side of the backing layer,
wherein the pressure-sensitive adhesive layer contains:
a drug component composed of tandospirone and/or a pharmaceutically acceptable salt thereof at 5 to 30 wt% based on the weight of the pressure-sensitive adhesive layer;
an acrylic-based polymer and/or styrene-based block copolymer; and
an organic acid and/or a salt thereof.

2. A patch according to claim 1,
wherein at least part of the tandospirone and at least part of the organic acid in the pressure-sensitive adhesive layer forms a pharmaceutically acceptable salt.

3. A patch according to claim 2,
wherein at least part of the drug component is crystallized.

4. A patch according to claim 3,
wherein the styrene-based block copolymer is a styrene-isoprene-styrene block copolymer.

5. A patch according to claim 4,
wherein the organic acid is acetic acid.

6. A patch according to claim 1,
wherein at least part of the drug component is crystallized.

7. A patch according to claim 6,
wherein the styrene-based block copolymer is a styrene-isoprene-styrene block copolymer.

8. A patch according to claim 7,
wherein the organic acid is acetic acid.

9. A patch according to claim 1,
wherein the styrene-based block copolymer is a styrene-isoprene-styrene block copolymer.

10. A patch according to claim 9,
wherein the organic acid is acetic acid.

11. A patch according to claim 1,
wherein the organic acid is acetic acid.

12. A patch according to any one of claims 1 to 11,
wherein the pressure-sensitive adhesive layer further contains a fatty acid ester and/or fatty acid amide.
